(19) Europäisches Patentamt
European Patent Office
Office européen des brevets

(11) Publication number: **0 502 448 A2**

# (12) EUROPEAN PATENT APPLICATION

(21) Application number: **92103486.4**

(22) Date of filing: **28.02.92**

(51) Int. Cl.5: **G01N 33/68**, G01N 33/564, G01N 33/574, //C07K15/00, C07K17/00,C12N15/12

(30) Priority: **01.03.91 JP 35911/91**

(43) Date of publication of application: **09.09.92 Bulletin 92/37**

(84) Designated Contracting States: **AT BE CH DE DK ES FR GB GR IT LI LU MC NL PT SE**

(71) Applicant: **KYOWA HAKKO KOGYO CO., LTD.**
**6-1, Ohtemachi 1-chome**
**Chiyoda-ku Tokyo-to(JP)**

(72) Inventor: **Satoh, Mitsuo**
**3-6-6, Asahi-machi**
**Machida-shi, Tokyo(JP)**
Inventor: **Miyaji, Hiromasa**
**2-11-2, Naruse**
**Machida-shi, Tokyo(JP)**
Inventor: **Sato, Seiji**
**2-6-3, Morinosato**
**Atsugi-shi, Kanagawa(JP)**
Inventor: **Itoh, Seiga**
**6-9-48, Aihara, Sagamihara-shi**
**Kanagawa(JP)**

(74) Representative: **Kinzebach, Werner, Dr. et al**
**Patentanwälte Reitstötter, Kinzebach und Partner Sternwartstrasse 4 Postfach 86 06 49**
**W-8000 München 86(DE)**

## (54) Method of assaying anti-ubiquitin antibody.

(57) A method of assaying anti-ubiquitin antibody is provided which comprises allowing anti-ubiquitin antibody in a sample to bind to polyubiquitin supported on a solid phase, then allowing a labeled anti-human immunoglobulin specific antibody to bind to the bound anti-ubiquitin antibody, and assaying the label on said specific antibody.

EP 0 502 448 A2

## FIELD OF THE INVENTION

This invention relates to a method of assaying antiubiquitin antibody.

## BACKGROUND OF THE INVENTION

Systemic lupus erythematosus (SLE) is a systemic inflammatory disease affecting a multiplicity of organs. While the causes thereof are not yet clear, it is a noninfectious and nontumor, but chronic and intractable disease. Serologically, various autoantibodies, in particular antinuclear antibodies, are detected, and immunological disorders, including cellular immunity, are observed. For the diagnosis of systemic lupus erythematosus, a method has been developed and is in use which assays an autoantibody, in particular an anti-DNA antibody, in sera of patients. However, this method can detect systemic lupus erythematosus only in about half of patients suffering from said disease, hence is not a highly sensitive method of diagnosis.

A method is known for determining anti-ubiquitin antibody, an autoantibody occurring with high frequency in sera of patients with systemic lupus erythematosus, by enzyme immunoassay using ubiquitin as an antigenic protein [Müller et al.: Proc. Natl. Acad. Sci. USA, 85, 8176 (1988)]. It is also known that a polypeptide comprising three repetitions of the peptide sequence shown under SEQ ID NO: 1 (hereinafter referred to as polyubiquitin) is present in living organisms [J. Biol. Chem., 260, 7609 (1985)]. However, these references neither disclose nor suggest the usability of the protein polyubiquitin as an antigenic protein.

Accordingly, it is an object of the invention to provide a diagnostic method for identifying patients with systemic lupus erythematosus with higher sensitivity by detecting anti-ubiquitin antibody, an autoantibody appearing, with higher frequency, in sera of patients with systemic lupus erythematosus.

## SUMMARY OF THE INVENTION

In accordance with the invention, a method of assaying anti-ubiquitin antibody in samples and an assay kit therefor are provided. The method comprises binding anti-ubiquitin antibody in a sample to polyubiquitin supported on a solid phase, then binding a labeled anti-human immunoglobulin specific antibody to the bound anti-ubiquitin antibody and determining the label on the bound anti-human immunoglobulin specific antibody.

## BRIEF DESCRIPTION OF THE DRAWINGS

Fig. 1 is a flow sheet illustrating the process for constructing the plasmid pLGC:Sc11.

Fig. 2 is a flow sheet illustrating the process for constructing the plasmid pLGC-x1.

Fig. 3 illustrates the reactivity of polyubiquitin as an antigenic protein for rabbit anti-ubiquitin polyclonal antibody in comparison with that of ubiquitin.

Fig. 4 shows the results of serodiagnosis according to the invention of systemic lupus erythematosus.

Fig. 5 schematically illustrates of cDNA synthesis by the Okayama-Berg process and construction of a recombinant plasmid containing said DNA.

Fig. 6 shows restriction enzyme maps of cDNAs contained in pLGA8, pLGB12 and pLGC15, respectively.

Fig. 7 shows a staining picture obtained by SDS-polyacrylamide gel electrophoresis of purified polyubiquitin.

## DETAILED DESCRIPTION OF THE INVENTION

A diagnostic method is provided for detecting anti-ubiquitin antibody, an autoantibody appearing in higher frequency in sera of patients with systemic lupus erythematosus. The method comprises contacting a sample to polyubiquitin and detecting the formation of a polyubiquitin/antiubiquitin antibody complex. Typically the polyubiquitin is supported on a solid phase, and the formation of a polyubiquitin/antiubiquitin antibody complex is determined by use of a labeled anti-human immunoglobulin specific antibody.

The polyubiquitin used in the subject invention is typically three repetitions of the peptide sequence shown under SEQ ID NO: 1. However, as will be obvious to the skilled person, variations of this polypeptide which result in higher sensitivity in the detection of anti-ubiquitin antibody, are also within the scope of the subject invention.

(1) Preparation of the polyubiquitin protein

The polyubiquitin protein can be obtained using the recombinant DNA technology as follows.

Cells are collected from a suspension of cells of the human erythroblastic cell line K562 (ATCC CCL 243) by centrifugation and solubilized in a solution containing guanidine thiocyanate. The solution is then layered onto a CsCl solution layer. The subsequent ultracentrifugation gives whole cytoplasmic RNA as a sediment. It is also possible to recover RNA alone by adding LiCl to the guanidine thiocyanate solubilization product to cause precipitation. The cell line K562 is a per se known and generally available cell line as described, for example, in Proc. Natl. Acad. Sci. USA, 76, 1293 (1979) or Blood, 45, 321 (1975).

The RNA thus obtained is dissolved in a high-concentration (e.g. 0.5 M) NaCl or KCl solution and the solution is passed through an oligo(dT)-cellulose column for adsorption of mRNA having polyadenylic acid [hereinafter, poly(A)]. The mRNA having poly(A) is eluted using water or a low-concentration salt solution such as 10 mM Tris-HCl buffer.

cDNA synthesis and insertion thereof into a vector are then carried out in accordance with the Okayama-Berg method [Mol. Cell. Biol., 2, 161 (1982)].

Thus, a vector primer is first synthesized. The plasmid pcDV1, for instance, is used as the vector. It is treated with KpnI in an appropriate solution, for example a solution containing 10 mM Tris-HCl buffer (pH 7.5), 6 mM $MgCl_2$ and 10 mM NaCl, for cleaving pcDV1 at the KpnI site. This DNA is incubated with terminal deoxynucleotidyl transferase (hereinafter, TdT) in a solution comprising, for example, 30 mM Tris-HCl buffer (pH 6.8), 140 mM sodium cacodylate, 1 mM $CoCl_2$, 0.1 mM dithiothreitol (hereinafter, DTT) and 0.25 mM deoxythymidine triphosphate (hereinafter, dTTP) at a certain temperature (e.g. 37° C) for a certain period (e.g. 20 minutes) for addition of about 60 thymidyl residues to both 3' ends of the vector DNA. The DNA is further cleaved with EcoRI in a solution containing, for example, 10 mM Tris-HCl buffer (pH 7.5), 6 mM $MgCl_2$ and 100 mM NaCl and a fragment of about 3.1 kilobases is recovered by fractionation, for example by low-gelling-temperature agarose gel electrophoresis [Lars Wieslander: Anal. Biochem., 98, 305 (1979); hereinafter, LGT method]. Said DNA is dissolved in a high-concentration (e.g. 0.5 M) NaCl or KCl solution, and the solution is passed through a polydeoxyadenylic acid-cellulose column for exclusive adsorption of the vector primer molecule having polythymidylic acid [hereinafter, poly(T)]. The vector primer molecule with poly(T) added thereto is isolated by elution using water or a low-concentration salt solution such as 10 mM Tris-HCl buffer.

A linker DNA is then synthesized. The pL1 DNA, for instance, is treated with PstI in an appropriate solution, for example a solution containing 10 mM Tris-HCl buffer (pH 7.5), 6 mM $MgCl_2$ and 50 mM NaCl, for cleaving pL1 at the PstI site. This DNA is treated in the same manner as in vector primer synthesis except that deoxyguanosine-5'-triphosphate (hereinafter, dGTP) is added in lieu of dTTP for addition of an oligo-dG chain comprising about 15 dGTP residues. The DNA is cleaved with HindIII in an appropriate solution, for example a solution containing 10 mM Tris-HCl buffer (pH 7.5), 6 mM $MgCl_2$ and 60 mM NaCl. A DNA fragment of about 0.5 kilobases is recovered using a DEAE paper following fractionation by agarose gel electrophoresis. A linker DNA is thus obtained.

cDNA synthesis is carried out using the poly(A)-RNA, vector primer and linker DNA obtained in the above manner. The poly(A)-RNA and vector primer are exposed to reverse transcriptase in a solution containing 50 mM Tris-HCl buffer (pH 8.3), 8 mM $MgCl_2$, 30 mM KCl and 0.3 mM DTT as well as deoxyadenosine 5'-triphosphate (hereinafter, dATP), dTTP, deoxycytidine 5'-triphosphate (hereinafter, dCTP) and dGTP (e.g. 2 mM each) at a certain temperature (e.g. 37° C) for a certain period (e.g. 40 minutes). An oligo-dC chain comprising about 15 dCTP residues is added to each 3' end of the thus-obtained RNA-DNA double strand under the same conditions as used for dT chain addition to the vector primer except that dCTP is used in lieu of dTTP. This DNA is cleaved with HindIII in a solution containing, for example, 10 mM Tris-HCl buffer (pH 7.5), 6 mM $MgCl_2$ and 60 mM NaCl. This DNA is mixed with the linker DNA prepared previously, and the mixture is incubated together with Escherichia coli-derived DNA ligase in a solution containing, for example, 20 mM Tris-HCl buffer (pH 7.5), 4 mM $MgCl_2$, 10 mM $(NH_4)_2SO_4$, 0.1 M KCl and 0.1 mM β- nicotinamide adenine dinucleotide (β-NAD) for a certain period (e.g. 16 hours) at an appropriate temperature (e.g. 12° C), whereby cyclization involving the cDNA and linker DNA is effected. To this reaction mixture are added dATP, dTTP, dGTP and dCTP (each to a final concentration of 40 μM), followed by addition of Escherichia coli-derived DNA ligase, Escherichia coli-derived DNA polymerase I and Escherichia coli-derived ribonuclease H (RNase) for converting the RNA portion to the corresponding DNA. A recombinant plasmid containing complete double-stranded DNA is thereby obtained.

Using the thus-obtained recombinant plasmid, an appropriate strain of Escherichia coli such as Escherichia coli C600SF8 [Cameron et al.: Proc. Natl. Acad. Sci. USA, 72, 3416 (1975)] is transformed by the method of Scott et al. [Katsuya Sigesada: Saibo Kogaku (Cell Technology), 2, 616 (1983)]. Since the recombinant plasmid obtained in the above manner contains the ampicillin resistance gene, Escherichia coli transformed therewith shows resistance to ampicillin.

A strain harboring the novel recombinant plasmid DNA containing a gene complementary to the polyubiquitin mRNA can be selected from among ampicillin-resistant (hereinafter, $Ap^r$) strains using the following techniques which are generally used. Thus, the transformants obtained in the above manner are immobilized on a nitrocellulose filter and treated with a synthetic DNA probe having a DNA sequence expectable from the amino acid sequence of human ubiquitin for association therewith, and one showing strong association is selected [Grunstein-Hogness method: Proc. Natl. Acad. Sci. USA, 72, 3961 (1975)]. The probe DNA can be synthesized by the conventional triester method [R. Crea et al.: Proc. Natl. Acad. Sci. USA, 75, 5765 (1978)]. The selection using the synthetic DNA can be further ensured by the method of Southern et al. [J. Mol. Biol., 98, 503 (1975)]. The plasmid is recovered from the strain showing strong association with the synthetic DNA and the whole base sequence of the translational region thereof is determined by the dideoxy sequencing method using the M13 phage [J. Messing et al.: Gene, 19, 269 (1985)]. A cDNA coding for polyubiquitin can thus be obtained.

Expression of polyubiquitin can be effected by inserting a DNA fragment coding for said polypeptide into an appropriate plasmid having DNA-expressing function.

Any DNA that codes for polyubiquitin can be used as the polyubiquitin cDNA. Suitable examples are a cDNA obtainable by reverse transcription from polyubiquitin-encoding mRNA by the recombinant DNA technology (cDNA prepared as described above and shown under SEQ ID NO: 14) and a polyubiquitin-encoding DNA obtainable from chromosomal DNA. More specifically, the plasmid pLGC15 produced by the present inventors can be used. A method of producing pLGC15 is described herein in an example.

Any plasmid that allows expression of the polyubiquitin-encoding DNA in Escherichia coli or animal cells can be used as the plasmid into which said DNA is to be inserted. Preferred plasmids are those which allow insertion of a foreign DNA at a site downstream from an appropriate promoter (e.g. trp or lac promoter) and in which the distance between the Shine-Dalgarno sequence and the initiation codon (ATG) is adequate, for example 6 to 18 base pairs. Specifically, the plasmid pKYP10, pKYP11 and pKYP12 constructed by the present inventors (U.S. Patent 4,686,191 corresponding to JP-A-58-110600), among others, are preferred plasmids (the term "JP-A" means an unexamined published Japanese patent application).

The following is an example in which a recombinant plasmid containing a polyubiquitin-encoding DNA is constructed by using pLGC15 as the polyubiquitin cDNA source and pKYP10 as the plasmid for insertion of said cDNA thereinto.

As shown in Fig. 1, pLGC15 is cleaved with XhoI and a DNA fragment of about 1.3 Kb is purified by the LGT method. Separately, pUC19 [C. Yanisch-Perron et al.: Gene, 33, 103 (1985)] is cleaved with SalI and a DNA fragment of about 2.7 Kb is purified by the LGT method. The thus-obtained DNA fragments are ligated together using T4 DNA ligase to give pLGC:Sc11. Then, as shown in Fig. 2, pLGC:Sc11 is subjected to partial digestion with BglII, which is followed by treatment with DNA polymerase I Klenow fragment (hereinafter, Klenow fragment) and cleavage with BamHI. A DNA fragment of about 950 bp is then purified by the LGT method. Separately, pGEL1 [Sekine et al.: Proc. Natl. Acad. Sci. USA, 82, 4306 (1985)] is cleaved with BamHI and HindIII and PstI, and a DNA fragment of about 1.7 Kb is purified by the LGT method. Further, separately, pKYP10 is cleaved with PstI and BanIII and a DNA fragment of about 1.1 Kb is purified by the LGT method. The thus-obtained three DNA fragments and the synthetic DNA shown in Fig. 2 [SEQ ID NO: 5, containing the initiation codon (ATG) and the first two bases (CA) of the triplet (CAG) coding for the second amino acid glutamine (Gln)] are subjected to ligation using T4 DNA ligase to give pLGC-x1. In Fig. 1 and Fig. 2, UL-C is the DNA region shown under SEQ ID NO: 14.

The reaction conditions to be employed in the above recombination techniques are generally as follows.

For restriction enzyme digestion of DNA, 0.1 to 20 µg of DNA is generally treated with 0.1 to 100 units (preferably 1 to 3 units per µg of DNA) of restriction enzyme in a reaction solution containing 2 to 200 mM (preferably 10 to 40 mM) Tris-HCl buffer (pH 6.0 to 9.5, preferably pH 7.0 to 8.0), 0 to 200 mM NaCl and 2 to 20 mM (preferably 5 to 10 mM) $MgCl_2$ at a temperature of 20 to 70 °C (the optimal temperature varies according to the restriction enzyme used) for 15 to 24 hours. The digestion reaction is terminated generally by heating at 55 to 75 °C for 5 to 30 minutes. The reaction may also be terminated by inactivating the restriction enzyme with such a reagent as phenol or diethyl pyrocarbonate.

The DNA fragments produced by restriction enzyme digestion can be purified by the LGT method mentioned above or by polyacrylamide gel electrophoresis.

The ligation of DNA fragments is carried out in a reaction solution containing 2 to 200 mM (preferably 10 to 40 mM) Tris-HCl buffer (pH 6.1 to 9.5, preferably pH 7.0 to 8.0), 2 to 20 mM (preferably 5 to 10 mM) $MgCl_2$, 0.1 to 10 mM (preferably 0.5 to 2.0 mM) ATP and 1 to 50 mM (preferably 5 to 10 mM) DTT in the presence of 0.3 to 100 units of T4 DNA ligase at 1 to 37°C (preferably 3 to 20°C) for 15 minutes to 72 hours (preferably 2 to 20 hours).

The recombinant plasmid DNA resulting from ligation is introduced into Escherichia coli by the transformation method of Cohen et al. [S. N. Cohen et al.: Proc. Natl. Acad. Sci. USA, 69, 2110 (1972)], as necessary.

The recombinant plasmid DNA is isolated from Escherichia coli harboring said DNA by the method to be described later in Example 1 or by the method of Birnboim et al. [H. C. Birnboim et al.: Nucleic Acids Res., 7, 1513 (1979)], for instance.

For confirming the base sequence of plasmid DNA, the plasmid DNA is cleaved with 1 to 10 kinds of restriction enzyme and the cleavage sites are examined by agarose gel electrophoresis or polyacrylamide gel electrophoresis. When further DNA base sequence determination is required, the dideoxy sequencing method [J. Messing: Gene, 19, 269 (1985)] should be used.

The desired recombinant plasmid DNA can be constructed under conditions such as mentioned above.

Polyubiquitin can be produced in the following manner. Escherichia coli C600SF8 is transformed with a polyubiquitin-encoding plasmid (e.g. pLGC-x1) and a strain of Escherichia coli that carries pLGC-x1, for instance, is selected from among $Ap^r$ colonies. The Escherichia coli strain carrying pLGC-x1 is cultivated in a medium, whereby polyubiquitin is formed in the culture. The thus-produced polyubiquitin is purified by using a cation exchange resin or by ultrafiltration, for instance. The medium to be used may be a synthetic medium or a natural medium provided that it is suited for the growth of Escherichia coli and the production of polyubiquitin.

Glucose, fructose, lactose, glycerol, mannitol, sorbitol and the like may be used as carbon sources, $NH_4Cl$, $(NH_4)_2SO_4$, Casamino acids, yeast extract, polypeptone, meat extract, Bacto-tryptone, corn steep liquor and the like as nitrogen sources, and $K_2HPO_4$, $KH_2PO_4$, NaCl, $MgSO_4$, vitamin $B_1$, $MgCl_2$ and the like as other nutrients.

The cultivation is carried out at pH 5.5 to 8.5 and at a temperature of 18 to 40°C with aeration and agitation. After 5 to 90 hours of cultivation, polyubiquitin is found accumulated as intracellular granules in cultured cells. Cells are harvested from the culture mass, disrupted by sonication and centrifuged, whereby polyubiquitin is extracted into the supernatant.

Since the polyubiquitin fraction extracted and separated from cells contains Escherichia coli-derived proteolytic enzymes, the protease inhibitors diisopropyl fluorophosphate (DFP), EDTA, L-transepoxysuccinylleucylamindo-4-guanidinobutane (E-64) and pepstatin are added to said fraction to give the final concentrations of 1 mM 1 mM, 0.02 mM and 0.01 mM, respectively, whereby the decomposition of polyubiquitin is suppressed. To the protease inhibitor-treated solution is added a weakly acidic buffer solution for solubilization of impurities. Polyubiquitin is recovered as a precipitate. The thus-recovered polyubiquitin is solubilized by adding a weakly basic buffer solution containing 6 M urea, for instance. The solubilized fraction is passed through a cation exchange resin column and effluent fractions are combined as a purified polyubiquitin fraction. Useful as the cation exchange resin are, for example Sephadex species (Pharmacia Fine Chemicals) and Toyopearl (Tosoh). The amino acid sequence of the thus-obtained polyubiquitin can be determined, for example, by combined use of an Applied Biosystems model 470A sequencer and a Spectra Physics high performance liquid chromatography.

(2) Assaying of anti-ubiquitin antibody

Anti-ubiquitin antibody in samples, in particular human sera, is assayed using the polyubiquitin obtained as described above under (1), as follows.

A 10 to 100 µg/ml solution of the polyubiquitin obtained as described under (1) is distributed in 5 to 100 µl portions into wells of a 96-well plate for EIA and allowed to stand at 4°C for 10 to 30 hours or at room temperature for 2 to 4 hours. The plate is washed with phosphate buffer (aqueous solution containing 1.83 g of disodium phosphate, 0.21 g of monopotassium phosphate and 7.65 g of NaCl per liter, pH 7.2; hereinafter, PBS), then 200 µl of PBS containing 1% bovine serum albumin (BSA) is added to each well, and the plate is further allowed to stand at 4°C for 10 hours or at room temperature for 2 hours. This plate is washed well with PBS and then 50 to 100 µl per well of a serum sample in 1- to 500-fold dilution is added. After standing at 4°C for 10 hours, or at room temperature for 2 hours, the plate is washed well with PBS.

Then, a solution (10 to 100 µg/ml) of a labeled antihuman immunoglobulin specific antibody is added in an amount of 50 to 100 µl/well, and the plate is allowed to stand at 4°C for 10 hours or at room temperature for 30 minutes to 4 hours. The label for anti-human immunoglobulin specific antibody may be enzymes, biotin, chemiluminescent substances, radioactive compounds and the like. It is particularly preferred to use an enzyme-labeled anti-human immunoglobulin specific antibody.

Examples of the enzyme-labeled anti-human immunoglobulin antibody which are useful include directly labeled commercial antibodies in which the enzyme is peroxidase, urease, alkaline phosphatease or β-galactosidase, for instance, and antibodies indirectly labeled via biotin-avidin bonding (avidin-biotin-enzyme conjugates). The latter antibodies are also commercially available.

After standing, the plate is washed well with PBS. Then, 50 to 100 µl of an enzyme substrate solution is added to each well. The plate is allowed to stand at room temperature for 10 to 30 minutes and the reaction is then terminated by adding 50 to 100 µl/well of a 5% sodium dodecyl sulfate (SDS) solution. The enzyme substrate solution is, for example, an ABTS substrate solution (prepared by adding, just prior to use, 1 µl/ml of hydrogen peroxide to a solution prepared by dissolving 550 mg of diammonium 2,2'-azinobis(3-ethylbenzothiazoline-6-sulfonate) in 1 liter of 0.1 M citrate buffer (pH 4.2)] when peroxidase-labeled anti-human immunoglobulin specific antibody is used; a solution containing 8 mg of bromocresol purple, 100 mg of urea and 0.2 mM EDTA per 100 ml and having a pH of 4.8, as a substrate for urease; or a 1 mg/ml solution of p-nitrophenyl phosphate in 0.1 M diethanolamine buffer (pH 9.6), as a substrate for alkaline phosphatase. After termination of the reaction, the $OD_{415}$ value is determined for each well and the amount of anti-ubiquitin antibody in the sample is calculated based on the intensity of color development. When a urease-labeled anti-human immunoglobulin antibody is used, the measurement is conducted as described in J. of Immunological Methods, 53, 187 (1982).

(3) Serodiagnosis of patients with systemic lupus erythematosus

The amounts of anti-ubiquitin antibody in sera from normal subjects as found by the assay method described above under (2) are compared with those in sera from patients with systemic lupus erythematosus as found in the same manner to thereby establish a normal range. A patient is judged as positive with respect to systemic lupus erythematosus when the amount of said antibody in the serum of said patient exceeds said normal range.

The following reference examples and working examples are further illustrative of the present invention.

EXAMPLE 1

Comparison between ubiquitin and polyubiquitin as antigenic proteins

A 15 µg/ml solution of ubiquitin (Sigma) or polyubiquitin obtained in Reference Example 6 (see below) was distributed in 50-µl portions into wells of a 96-well plate for EIA (Nunc) and allowed to stand at 4°C for 10 hours. The plate was washed with PBS, 200 µl of a PBS solution containing 1% BSA was added to each well, and the plate was allowed to stand for 1 hour and then washed well with PBS. To this plate were added solutions of rabbit anti-ubiquitin polyclonal antibody [A. L. Haas et al.: J. Biol. Chem., 260, 12464 (1985)] having concentrations of 10, 1, 0.1, 0.01 and 0.001 µg/ml, respectively, in an amount of 50 µl/well. After 2 hours of standing at room temperature, the plate was washed well with PBS. Then, 50 µl of a 10 µg/ml solution of peroxidase-labeled anti-rabbit immunoglobulin specific antibody (Dako) was added to each well. The plate was allowed to stand at room temperature for 2 hours and then washed well with PBS. Furthermore, 50 µl of the ABTS substrate solution was added to each well, and the reaction was allowed to proceed at room temperature for 30 minutes and then terminated by addition of 100 µl/well of a 5% SDS solution. For each well, the color development ($OD_{415}$) was measured using an Immunoreader NJ-2000 absorptiometer (Nippon Intermed). As a result, as shown in Fig. 3, the use of polyubiquitin as the antigenic protein resulted in more intense color development as compared with the case of ubiquitin. It was thus revealed that anti-ubiquitin antibodies can be assayed with higher sensitivity using polyubiquitin as the antigenic protein as compared with the case where ubiquitin is used.

EXAMPLE 2

Serodiagnosis of systemic lupus erythematosus (SLE) in accordance with the invention

A 10 µg/ml solution of ubiquitin (Sigma) or polyubiquitin obtained in Reference Example 6 was distributed in 50-µl portions into wells of a 96-well EIA plate (Nunc). The plate was allowed to stand at 4°C for 10 hours and then washed with PBS. A PBS solution containing 1% BSA was distributed in 200-µl portions into the wells, and the plate was allowed to stand for 1 hour and then washed well with PBS. To this plate were added 10 serum samples from normal humans and 34 serum samples from patients with systemic lupus erythematosus, each 200-fold diluted with a PBS solution containing 0.05% Tween-20 and 1% BSA and in an amount of 50 µl/well. The plate was allowed to stand at room temperature for 2 hours and then washed well with PBS. Then, 50 µl of a 10 µg/ml solution of peroxidase-labeled anti-human immunoglobulin specific antibody (Dako) was added to each well. The plate was allowed to stand at room temperature for 2 hours and then washed well with PBS. Furthermore, 50 µl of the ABTS substrate solution was added to each well and the reaction was allowed to proceed at room temperature for 30 minutes and then terminated by addition of 100 µl/well of a 5% SDS solution. For each well, the color development ($OD_{415}$) was measured using an Immunoreader NJ-2000 absorptiometer (Nippon Intermed). The results thus obtained are shown in Fig. 4. When ubiquitin was used as the antigenic protein, the number of positive cases in which the $OD_{415}$ value exceeded 0.06 was zero out of the 10 serum samples from normal subjects while that was 25 out of the 34 serum samples from patients with systemic lupus erythematosus. When polyubiquitin was used as the antigenic protein, the number of positive cases in which the $OD_{415}$ value exceeded 0.1 was zero out of the 10 serum samples from normal subjects while that was 31 out of the 34 serum samples from patients with systemic lupus erythematosus. This indicates that the serodiagnostic method of the present invention is superior to the case with ubiquitin and can detect about 90% of patients with systemic lupus erythematosus.

REFERENCE EXAMPLE 1

Preparation of poly(A) RNA from the cell line K562

Poly(A)-containing RNA was prepared from the human myelocytic leukemia cell line K562 (ATCC CCL 243) by the guanidine thiocyanate-lithium chloride method [Cathala et al.: DNA, 2, 329 (1983)] as follows.

The cell line K562 was inoculated into 1 liter of a protein-free medium prepared by mixing the basal media HAM-F10 medium (Flow Laboratories) and Dulbecco's MEM (Nissui Pharmaceutical) in a ratio of 3:1 and supplementing the mixture with 5 mM pyruvic acid, 1.25 x $10^{-7}$ M selenious acid, 1 mg/ml galactose, 4 mM glutamine, 25 U/ml penicillin, 25 µg/ml streptomycin and 0.01% sodium bicarbonate, at an inoculum size of 8 x $10^5$ cells/ml, and cultivated in a spinner culture bottle at 37° C for 3 days.

Then, a part (250 ml) of the resultant cell suspension was subjected to centrifugation (1,100 x g, 4° C, 10 minutes) for harvesting cells. The cells were washed with 80 ml of phosphate buffer (pH 7.0) and then solubilized in 10 ml of a solution comprising 5 M guanidine thiocyanate, 10 mM EDTA, 50 mM Tris-HCl buffer (pH 7.0) and 8% (v/v) 2-mercaptoethanol using a vortex mixer. This solubilization mixture was transferred to a centrifuge tube, 80 ml of 4 M LiCl was added, and the mixture was stirred, allowed to stand at 4° C for 20 hours and then centrifuged using a Hitachi RPR10 rotor (9,000 rpm, 90 minutes). RNA was recovered as a sediment. The sediment RNA was suspended in 50 ml of a solution comprising 4 M urea and 2 M lithium chloride and the suspension was centrifuged using a Hitachi RPR10 rotor at 9,000 rpm for 60 minutes, and RNA was recovered again as a sediment. The sediment RNA was dissolved in 10 ml of a solution comprising 0.1% sodium lauryl sulfate, 1 mM EDTA and 10 mM Tris-HCl buffer (pH 7.5) and, after phenol-chloroform extraction, recovered by ethanol precipitation. The thus-obtained RNA (about 2.5 mg) was dissolved in 1 ml of a solution comprising 10 mM Tris-HCl buffer (pH 8.0) and 1 mM EDTA. The solution was incubated at 65° C for 5 minutes, and 0.1 ml of 5 M NaCl was added. The mixture was applied to an oligo(dT)-cellulose column (P-L Biochemicals) (column volume 0.5 ml) for chromatography. The poly-(A)-containing mRNA thus adsorbed on said column was eluted with a solution comprising 10 mM Tris-HCl buffer (pH 7.5) and 1 mM EDTA to give about 100 µg of poly(A)-containing mRNA.

REFERENCE EXAMPLE 2

cDNA synthesis and insertion of the polyubiquitin cDNA into a vector

The Okayama-Berg method [Okayama & Berg: Mol. Cell. Biol., 2, 161 (1982)] was followed for cDNA synthesis and construction of a recombinant plasmid containing the cDNA as an insert. The process is outlined in Fig. 5.

pcDV1 (400 µg; Pharmacia) [Okayama & Berg: Mol. Cell. Biol., 3, 280 (1983)] was added to 300 µl of a solution comprising 10 mM Tris-HCl buffer (pH 7.5), 6 mM $MgCl_2$ and 10 mM NaCl and, after further addition of 500 units of KpnI, the digestion reaction was allowed to proceed at 37° C for 6 hours for cleaving the plasmid at the KpnI site. After phenol-chloroform extraction, DNA was recovered by ethanol precipitation. About 200 µg of the KpnI-cleaved DNA was added to 200 µl of a solution prepared by adding dTTP, to a concentration of 0.25 mM, to a buffer solution comprising 40 mM sodium cacodylate, 30 mM Tris-HCl buffer (pH 6.8), 1 mM $CaCl_2$ and 0.1 mM DTT (hereinafter, TdT buffer) and, after further addition of 81 units of TdT (P-L Biochemicals), the poly(dT) addition reaction was carried out at 37° C for 11 minutes, whereby a poly(dT) chain, about 67 bases in length, was added to each 3' terminus of the KpnI cleavage site of pcDV1. Following phenol-chloroform extraction, about 100 µg of the poly(dT) chain-containing pcDV1 DNA was recovered from said solution by ethanol precipitation. Said DNA was added to 150 µl of a buffer solution comprising 10 mM Tris-HCl buffer (pH 7.5), 6 mM $MgCl_2$ and 100 mM NaCl, 360 units of EcoRI was further added, and the digestion reaction was conducted at 37° C for 2 hours. The reaction mixture was treated by the LGT method and a DNA fragment of about 3.1 Kb was recovered. About 60 µg of poly(dT)-tailed pcDV1 was thus obtained. Said DNA was dissolved in 500 µl of a solution comprising 10 mM Tris-HCl buffer (pH 8.0) and 1 mM EDTA. The solution was incubated at 65° C for 5 minutes and then quenched with ice, and 50 µl of 5 M NaCl was added. The mixture was subjected to chromatography on an oligodeoxyadenylic acid-modified cellulose column (Collaborative Research). DNA having a sufficiently long poly(dT) chain was adsorbed on the column. It was eluted with a solution comprising 10 mM Tris-HCl buffer (pH 8.0) and 1 mM EDTA, whereby 27 µg of poly(dT)-tailed pcDV1 (hereinafter, vector primer) was obtained.

A linker DNA was then prepared.

About 14 µg of pL1 (Pharmacia) [Okayama & Berg: Mol. Cell. Biol, 3, 280 (1983)] was added to 200 µl of a buffer solution comprising 10 mM Tris-HCl buffer (pH 7.5), 6 mM $MgCl_2$ and 50 mM NaCl, 50 units of PstI was further added, and the digestion reaction was carried out at 37° C for 4 hours for cleavage of the pL1 DNA at the PstI site. The reaction mixture was subjected to phenol-chloroform extraction and then about 13 µg of the PstI-cleaved pL1 DNA was recovered by ethanol precipitation. About 13 µg of said DNA was added to 50 µl of a solution prepared by adding dGTP to TdT buffer to a final concentration of 0.25 mM, 54 units of TdT was further added, and the mixture was incubated at 37° C for 13 minutes for addition of a (dG) chain about 14 bases in length to each 3' end of the PstI cleavage site of pL1. The DNA was recovered by ethanol precipitation following phenol-chloroform extraction and added to 100 µl of a buffer solution comprising 10 mM Tris-HCl (pH 7.5), 6 mM $MgCl_2$ and 60 mM NaCl, 80 units of HindIII was further added, and the mixture was incubated at 37° C for 3 hours for cleavage of the pL1 DNA at the HindIII site. The reaction mixture was fractionated by agarose gel electrophoresis and a DNA fragment of about 0.5 Kb was recovered by the DEAE-paper method [Dretzen et al.: Anal. Biochem., 112, 295 (1981)]. In this manner, an oligo(dG)-tailed linker DNA (hereinafter, linker DNA for short) was obtained.

About 2 µg of the poly(A)-RNA and about 1.4 µg of the vector primer each prepared as described above were dissolved in 22.3 µl of a solution comprising 50 mM Tris-HCl buffer (pH 8.3), 8 mM $MgCl_2$, 30 mM KCl, 0.3 mM DTT, 2 mM each dNTP (dATP, dTTP, dGTP and dCTP) and 10 units of ribonuclease inhibitor (P-L Biochemicals), 10 units of reverse transcriptase (Seikagaku Corp.) was added, and the mixture was incubated at 41° C for 90 minutes for the synthesis of a DNA complementary to the mRNA. The reaction mixture was subjected to phenol-chloroform extraction followed by ethanol precipitation, whereby the vector primer DNA was recovered with an RNA-DNA double strand added thereto. Said DNA was dissolved in 20 µl of TdT buffer containing 66 µM dCTP and 0.2 µg of poly(A), 14 units of TdT was added, and the mixture was incubated at 37° C for 2 minutes for 3'-terminal addition of a (dC) chain (20 bases long) to the cDNA. The reaction mixture was subjected to phenol-chloroform extraction and then ethanol precipitation, whereby the (dC) chain-carrying cDNA-vector primer DNA was recovered. Said DNA was dissolved in 400 µl of a solution comprising 10 mM Tris-HCl buffer (pH 7.5), 6 mM $MgCl_2$ and 60 mM NaCl, 20 units of HindIII was added, and the mixture was incubated at 37° C for 2 hours for cleavage at the HindIII site. The reaction mixture was subjected to phenol-chloroform extraction and then to ethanol precipitation, whereby 0.5 picomole of a (dC) chain-carrying cDNA-vector primer DNA was obtained. Said DNA (0.2 picomole) and 0.4 picomole of the linker DNA mentioned above were dissolved in 100 µl of a solution comprising 10 mM Tris-HCl buffer (pH 7.5), 0.1 M NaCl and 1 mM EDTA, and the solution was incubated at 65° C for 10 minutes, then at 42° C for 25 minutes and further at 0° C for 30 minutes. The total volume of the reaction mixture was made 1,000 µl while the composition thereof was adjusted as follows: 20 mM Tris-HCl buffer (pH 7.5), 4 mM $MgCl_2$, 10 mM $(NH_4)_2SO_4$, 0.1 M KCl and 0.1 mM β-NAD. After addition of 25 units of Escherichia coli-derived ligase (New England Biolabs), said reaction mixture was incubated at 11° C for 18 hours. To the reaction mixture were added dNTPs and β-NAD for composition adjustment as follows: 40 µM each dNTP and 0.15 mM β-NAD. Ten units of Escherichia coli-derived DNA ligase, 20 units of Escherichia coli-derived polymerase I (P-L Biochemicals) and 10 units of Escherichia coli-derived ribonuclease H (P-L Biochemicals) were added, and the resultant mixture was incubated at 12° C for 1 hour and then at 25° C for 1 hour. The above reaction procedure caused cyclization of the cDNA-containing recombinant DNA and substitution of DNA for the RNA portion of the RNA-DNA double strand to give a recombinant plasmid in the complete double-stranded DNA form.

REFERENCE EXAMPLE 3

Selection of a polyubiquitin cDNA-containing recombinant DNA

The recombinant plasmid obtained in Reference Example 2 was used to transform Escherichia coli C600SF8 [Cameron et al.: Proc. Natl. Acd. Sci. USA, 72, 3416 (1975); deposited by the instant applicant with the Fermentation Research Institute, Agency of Industrial Science and Technology of 1-3, Higashi 1-chome, Tsukuba-shi, Ibaraki, Japan under the deposit number FERM BP-1070 as of May 27, 1986] by the method of Scott et al. [Katsuya Shigesada: Saibo Kogaku, 2, 616 (1983)]. About 10 thousand colonies obtained were immobilized on a nitrocellulose filter. Eight strains were selected which had strongly associated with a probe prepared by labeling a synthetic DNA corresponding to the 1st to 15th (from the N terminus) amino acids of the ubiquitin precursor encoded by the human ubiquitin precursor gene isolated by Wiborg et al. [O. Wiborg et al.: EMBO J., 4, 755 (1985)], namely the synthetic DNA shown under SEQ ID NO: 2 with $^{32}P$, at 45° C and with a probe prepared by labeling a synthetic DNA mixture corresponding to the 30th to 35th (from the N terminus) amino acids of said ubiquitin precursor, namely the synthetic DNA mixture shown under SEQ ID NO: 3 (the 3rd base being C, T or A, the 6th base being A or G, the 9th base being T or C, the 12th base being G or A and the 15th base being A or G; hence a mixture of 48 synthetic DNAs resulting from possible combinations), with $^{32}P$, at 40° C [Grunstein-Hogness method; Proc. Natl. Acad. Sci. USA, 72, 3961 (1975)]. The plasmids isolated from these were named pLGA8, pLGA9, pLGA11, pLGA13, pLGB12, pLGB16, pLGB19 and pLGC15, respectively. The eight plasmids obtained were digested with various restriction enzymes and cleavage maps were prepared for the respective cDNA portions. Based on the locations of restriction enzyme sites, the plasmids obtained could be classified into three groups, namely the group of pLGA8, pLGA9, pLGA11 and pLGA13 the group of pLGB12, pLGB16 and pLGB19, and pLGC15. Restriction enzyme maps for the respective groups are shown in Fig. 6.

The plasmids pLGA8, pLGB12 and pLGC15 presumably containing the respective cDNAs in a substantially full length form were selected from the respective three groups and the whole base sequence of the translational region of each plasmid was determined by the dideoxy sequencing method using the M13 phage [J. Messing et al.: Gene, 19, 269 (1985)]. The amino acid sequences deducible from the cDNAs are shown under SEQ ID NO: 10 (for pLGA8), SEQ ID NO: 12 (for pLGB12) and SEQ ID NO: 14 (for pLGC15). It was found that each sequence contains the amino acid sequence corresponding to human ubiquitin as a part of its structure and that pLGC15 codes for polyubiquitin comprising three ubiquitin sequences joined together.

REFERENCE EXAMPLE 4

Construction of the recombinant plasmid pLGC:Sc11

An Escherichia coli C600SF8 transformant carrying the plasmid pLGC15 (4.2 Kb) obtained in Reference Example 3 was cultivated and the pLGC15 DNA was prepared from cultured cells by the conventional method [Maniatis et al.: Molecular Cloning (1982), Cold Spring Harbor Laboratory]. 3 $\mu$g of the pLGC15 DNA obtained was dissolved in a total volume of 60 $\mu$l of solution containing 10 mM Tris-HCl buffer (pH 7.5), 7 mM $MgCl_2$, 6 mM 2-mercaptoethanol and 100 mM NaCl (hereinafter, Y-100 buffer), 6 units of the restriction enzyme XhoI was added, and the cleavage reaction was carried out at 37° C for 2 hours. About 0.5 $\mu$g of a polyubiquitin protein (UL-C) gene-containing DNA fragment (XhoI fragment) of about 1.3 Kb was obtained from the reaction mixture by the LGT method.

Separately, 2 $\mu$g of pUC19 (2.7 Kb) was dissolved in a total volume of 40 $\mu$l of a solution containing 10 mM Tris-HCl buffer (pH 7.5), 7 mM $MgCl_2$, 6 mM 2-mercaptoethanol and 175 mM NaCl, 10 units of the restriction enzyme SalI was added, and the cleavage reaction was conducted at 37° C for 2 hours. About 1.0 $\mu$g of a DNA fragment (SalI fragment) of about 2.7 Kb was obtained from the reaction mixture by the LGT method.

Then, 0.4 $\mu$g of the pLGC15-derived XhoI fragment (about 1.3 Kb) and 0.3 $\mu$g of the pUC19-derived SalI fragment (about 2.7 Kb), each prepared as described above, were dissolved in a total volume of 20 $\mu$l of a buffer solution containing 20 mM Tris-HCl (pH 7.6), 10 mM $MgCl_2$, 10 mM DTT and 1 mM ATP (hereinafter, T4 ligase buffer), 2 units of T4 DNA ligase (Takara Shuzo) was further added to the mixed solution, and the ligation reaction was conducted at 4° C for 18 hours.

The recombinant plasmid-containing reaction mixture was used to transform Escherichia coli C600SF8 to give $Ap^r$ colonies. The plasmid DNA was isolated and purified from one of the transformant strains by a per se known method [H. C. Birnboim et al.: Nucleic Acids Res., 7, 1513 (1979)]. The structure of the plasmid obtained was confirmed by cleavage with the restriction enzymes SalI and BamHI followed by polyacrylamide gel electrophoresis. This plasmid is called pLGC:Sc11.

REFERENCE EXAMPLE 5

Construction of the recombinant plasmid pLGC-x1

An Escherichia coli C600SF8 transformant carrying the plasmid pLGC:Sc11 (4.0 Kb) obtained in Reference Example 4 was cultivated and the pLGC:Sc11 DNA was prepared from cultured cells by a conventional method. The pLGC:Sc11 DNA obtained (10 µg) was dissolved in 100 µl of Y-100 buffer, 10 units of the restriction enzyme Bg1II was added, and the cleavage reaction was conducted at 37° C for 1 hour (the pLGC:Sc11 DNA was partially cleaved under these conditions). Following phenol extraction and chloroform extraction, ethanol precipitation was carried out, whereby about 8.0 µg of a DNA fraction was purified and recovered. About 8.0 µg of this 4.0-Kb DNA fragment was dissolved in a solution containing 50 mM Tris-HCl buffer (pH 7.8), 7 mM $MgCl_2$ and 6 mM 2-mercaptoethanol, dATP, dTTP, dCTP and dGTP were added each to a concentration of 1 mM (total volume 160 µl), 5 units of Klenow fragment (Takara Shuzo) was further added, and the reaction was conducted at room temperature for 1 hour. Following phenol extraction and chloroform extraction, ethanol precipitation was carried out to give about 6.0 µg of a DNA fragment. The DNA fragment thus recovered (6.0 µg) was dissolved in a total volume of 120 µl of Y-100 buffer, 12 units of BamHI was added, and the cleavage reaction was carried out at 37° C for 3 hours. About 0.1 µg of a DNA fragment [BglII (partial cleavage)-Klenow fragment-BamHI fragment] of about 950 bp containing the whole region of the UL-C polypeptide gene except a part of that portion corresponding to the N terminus of said polypeptide was recovered from the reaction mixture by the LGT method.

Separately, 2 µg of pGEL1 was dissolved in 40 µl of Y-100 buffer, 4 units each of the restriction enzymes HindIII, PstI and BamHI were added, and the cleavage reaction was conducted at 37° C for 3 hours. About 0.7 µg of a 1.7-Kb DNA fragment (PstI-BamHI fragment) containing the lipoprotein-derived terminator was recovered from the reaction mixture by the LGT method.

Further, 3 µg of the plasmid pKYP10 prepared by the method described in U.S. Patent 4,686,191 corresponding to JP-A-58-110600 was dissolved in Y-100 buffer, 6 units each of the restriction enzymes BanIII (Toyobo) and PstI were added, and the cleavage reaction was carried out at 37° C for 3 hours. About 0.6 µg of a DNA fragment of about 1.1 Kb (BanIII-PstI fragment) containing the tryptophan promoter (Ptrp) was recovered from the reaction mixture by the LGT method.

Further, separately, a DNA linker comprising the strands shown under SEQ ID NO: 5 and SEQ ID NO: 6 was synthesized for providing ATG [For the N-terminal Met of the polyubiquitin peptide (UL-C)] and the first two bases (CA) of the CAG codon (for the second amino acid Gln of said peptide), for adjusting the distance between the SD sequence downstream from Ptrp and said ATG to an appropriate length of 6 to 18 bp, and for other reasons. First, the 13-mer and 15-mer single-stranded DNAs were synthesized by the conventional phosphotriester method. Each 20 picomoles of the 13-mer and 15-mer were dissolved in a total volume of 40 µl of a solution containing 50 mM Tris-HCl buffer (pH 7.5), 10 mM $MgCl_2$, 5 mM DTT, 0.1 mM EDTA and 1 mM ATP, 1 unit of T4 polynucleotide kinase (Takara Shuzo) was added, and the phosphorylation was carried out at 37° C for 60 minutes.

Then, 0.1 µg of the pLGC:Sc11-derived BglII (partial cleavage)-Klenow fragment-BamHI fragment (about 950 bp), 0.1 µg of the pGEL1-derived PstI-BamHI fragment (about 1.7 Kb) and 0.3 µg of the BanIII-PstI fragment (about 1.1 Kb) of the expression vector pKYP10, each obtained as described above, were dissolved in 20 µl of T4 ligase buffer, and about 1 picomole of the above-mentioned DNA linker was added to the mixed solution. Further, 50 units of T4 DNA ligase was added to the mixed solution, and the ligation reaction was conducted at 4° C for 18 hours.

The recombinant plasmid-containing reaction mixture was used to transform Escherichia coli C600SF8 to give $Ap^r$ colonies. The plasmid was recovered from cultured cells derived from one of these colonies. The structure of the plasmid obtained was confirmed by cleavage with the restriction enzymes EcoRI, BglII and BamHI followed by polyacrylamide gel electrophoresis. This plasmid is called pLGC-x1. That the base sequence of pLGC-x1 in the vicinity of HindIII was as shown under SEQ ID NO: 7 was confirmed by the dideoxy sequencing method.

REFERENCE EXAMPLE 6

Production of the polyubiquitin peptide (UL-C) in an Escherichia coli transformant harboring pLGC-x1 and purification thereof

An Escherichia coli C600SF8 transformant carrying the recombinant plasmid pLGC-x1 obtained in Reference Example 5 was cultured in LG medium (prepared by dissolving 10 g of Bactotryptone, 5 g of yeast extract, 5 g of NaCl and 1 g of glucose in 1 liter of water and adjusting the pH to 7.0 with NaOH) at 37°C for 18 hours. Then, 50 ml of the culture was inoculated into 1 liter of MCG medium (containing 0.6% $Na_2HPO_4$, 0.3% $KH_2PO_4$, 0.5% NaCl, 0.5% Casamino acids, 1 mM $MgSO_4$ and 4 µg/ml vitamin $B_1$; pH 7.2) supplemented with 25 µg/ml of tryptophan and 50 µg/ml of ampicillin. Cultivation was conducted at 30°C for 4 to 8 hours, then 3β-indoleacrylic acid (tryptophan operon inducer) was added to a concentration of 10 µg/ml, and cultivation was further continued for 2 to 12 hours. Cells were collected by centrifuging the culture at 8,000 rpm for 10 minutes using a Hitachi RPR10 rotor and again suspended in PBS for washing. The cells washed were suspended in 100 ml of distilled water and sonicated at 0°C (treated for 10 minutes using Branson Sonic Power Company's Sonifier Cell Disruptor 200 in the mode OUTPUT 2). The resultant mass was centrifuged at 5,000 rpm for 30 minutes using a Hitachi RPRW20-2-103 rotor for separation and extraction of polyubiquitin into the supernatant. To this supernatant were added the protease inhibitors DFP, E-64 and pepstatin to final concentrations of 1 mM, 0.02 mM and 0.01 mM, respectively (the protease inhibitor solutions added each had a concentration 1,000 times the final concentration). Further, 9 volumes of 11 mM acetate buffer solution (pH 4.5) containing 6.6 M urea, 1.1% 2-mercaptoethanol and 1.1 mM EDTA was added. The resultant mixture was shaked at 4°C for 10 hours and then centrifuged at 8,000 rpm for 60 minutes using a Hitachi RPR10 rotor. The sediment thus recovered was dissolved in 1 liter of a 10 mM Tris-HCl solution (pH 8.0) containing 6 M urea, 1% 2-mercaptoethanol and 1 mM EDTA and the solution was passed through a DEAE-Toyopearl (Tosoh) column (50 mm in diameter, 50 cm in length) equilibrated with the same buffer at a flow rate of 1.8 ml/min. The effluent fraction that had passed through the column without undergoing adsorption was collected and used as a purified sample. The results of SDS-polyacrylamide gel electrophoresis of the thus-purified polyubiquitin are shown in Fig. 7. The SDS-polyacrylamide gel electrophoresis was performed by the method of Laemmli et al. [U.K. Laemmli: Nature 227, 680 (1970)] using a gradient SDS-PAG plate (10/20) for electrophoresis (Daiichi Pure Chemicals) and adding 0.5 µg of the purified sample to the groove. The molecular weight markers used were those of Bio-Rad's SDS-PAGE Standard (Low). The purified sample was detected as a single band in the vicinity of a molecular weight of about 26,000 as expected from the sequence of the DNA. The amino acid sequence of this purified sample was further determined for the N-terminal 30 amino acids by combined use of an Applied Biosystems model 470A sequencer and a Spectra Physics high-performance liquid chromatograph. The sequence shown under SEQ ID NO: 9 was thus revealed.

In accordance with the present invention, serodiagnosis of patients with systemic lupus erythematosus can be performed in a simple and efficient manner.

While the invention has been described in detail and with reference to specific examples thereof, it will be apparent to one skilled in the art that various changes and modifications can be made therein without departing from the spirit and scope thereof.

SEQUENCE LISTING

(1) GENERAL INFORMATION:

(i) APPLICANT: SATOH, MITSUO
MIYAJI, HIROMASA
SATO, SEIJI
ITOH, SEIGA

(ii) TITLE OF INVENTION: METHOD OF ASSAYING ANTI-UBIQUITIN ANTIBODY

(iii) NUMBER OF SEQUENCES: 15

(iv) CORRESPONDENCE ADDRESS:
(A) ADDRESSEE: NIXON & VANDERHYE P.C.
(B) STREET: 2200 CLARENDON BOULEVARD
(C) CITY: ARLINGTON
(D) STATE: VIRGINIA
(E) COUNTRY: U.S.A.
(F) ZIP: 22201-3360

(v) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.24

(vi) CURRENT APPLICATION DATA:
(A) APPLICATION NUMBER:
(B) FILING DATE:
(C) CLASSIFICATION:

(ix) TELECOMMUNICATION INFORMATION:
(A) TELEPHONE: (703)875-0400
(B) TELEFAX: (703)525-3468
(C) TELEX: 200797

(2) INFORMATION FOR SEQ ID NO:1:

(i) SEQUENCE CHARACTERISTICS:
(A) LENGTH: 76 amino acids
(B) TYPE: amino acid
(D) TOPOLOGY: linear

(ii) MOLECULE TYPE: protein

(iii) HYPOTHETICAL: N

(vi) ORIGINAL SOURCE:
(A) ORGANISM: Homo sapiens
(F) TISSUE TYPE: Blood

(ix) FEATURE:
(A) NAME/KEY: Protein
(B) LOCATION: 1..76
(D) OTHER INFORMATION: /label= UBIQUITIN

(xi) SEQUENCE DESCRIPTION: SEQ ID NO:1:

```
Met Gln Ile Phe Val Lys Thr Leu Thr Gly Lys Thr Ile Thr Leu Glu
1               5                   10                  15
```

```
Val Glu Pro Ser Asp Thr Ile Glu Asn Val Lys Ala Lys Ile Gln Asp
            20                  25                  30

Lys Glu Gly Ile Pro Pro Asp Gln Gln Arg Leu Ile Phe Ala Gly Lys
        35                  40                  45

Gln Leu Glu Asp Gly Arg Thr Leu Ser Asp Tyr Asn Ile Gln Lys Glu
    50                  55                  60

Ser Thr Leu His Leu Val Leu Arg Leu Arg Gly Gly
65                  70                  75
```

(2) INFORMATION FOR SEQ ID NO:2:

(i) SEQUENCE CHARACTERISTICS:
(A) LENGTH: 45 base pairs
(B) TYPE: nucleic acid
(C) STRANDEDNESS: single
(D) TOPOLOGY: linear

(ii) MOLECULE TYPE: cDNA to mRNA

(iii) HYPOTHETICAL: N

(ix) FEATURE:
(A) NAME/KEY: CDS
(B) LOCATION: 1..45
(D) OTHER INFORMATION: /product= "UBIQUITIN PRECURSOR"
/note= "THE 1ST TO 15TH AMINO ACIDS OF UBIQUITIN PRECURSOR"

(xi) SEQUENCE DESCRIPTION: SEQ ID NO:2:

```
ATG CAG ATC TTC GTG AAG ACC CTG ACT GGT AAG ACC ATC ACC CTC          45
Met Gln Ile Phe Val Lys Thr Leu Thr Gly Lys Thr Ile Thr Leu
  1               5                  10                  15
```

(2) INFORMATION FOR SEQ ID NO:3:

(i) SEQUENCE CHARACTERISTICS:
(A) LENGTH: 15 amino acids
(B) TYPE: amino acid
(D) TOPOLOGY: linear

(ii) MOLECULE TYPE: protein

(xi) SEQUENCE DESCRIPTION: SEQ ID NO:3:

```
Met Gln Ile Phe Val Lys Thr Leu Thr Gly Lys Thr Ile Thr Leu
  1               5                  10                  15
```

(2) INFORMATION FOR SEQ ID NO:4:

(i) SEQUENCE CHARACTERISTICS:
(A) LENGTH: 17 base pairs
(B) TYPE: nucleic acid
(C) STRANDEDNESS: single
(D) TOPOLOGY: linear

(ii) MOLECULE TYPE: cDNA to mRNA

(iii) HYPOTHETICAL: N

(ix) FEATURE:
(A) NAME/KEY: CDS
(B) LOCATION: 1..15
(D) OTHER INFORMATION: /product= "UBIQUITIN PRECURSOR"
/note= "THE 30TH TO 35TH AMINO ACIDS OF UBIQUITIN PRECURSOR"

(xi) SEQUENCE DESCRIPTION: SEQ ID NO:4:

ATHCARGAYA ARGARGG 17

(2) INFORMATION FOR SEQ ID NO:5:

(i) SEQUENCE CHARACTERISTICS:
(A) LENGTH: 15 base pairs
(B) TYPE: nucleic acid
(C) STRANDEDNESS: single
(D) TOPOLOGY: linear

(ii) MOLECULE TYPE: cDNA to mRNA

(iii) HYPOTHETICAL: N

(xi) SEQUENCE DESCRIPTION: SEQ ID NO:5:

CGATAAGCTT ATGCA 15

(2) INFORMATION FOR SEQ ID NO:6:

(i) SEQUENCE CHARACTERISTICS:
(A) LENGTH: 13 base pairs
(B) TYPE: nucleic acid
(C) STRANDEDNESS: single
(D) TOPOLOGY: linear

(ii) MOLECULE TYPE: cDNA to mRNA

(iii) HYPOTHETICAL: N

(xi) SEQUENCE DESCRIPTION: SEQ ID NO:6:

TGCATAAGCT TAT 13

(2) INFORMATION FOR SEQ ID NO:7:

(i) SEQUENCE CHARACTERISTICS:
(A) LENGTH: 25 base pairs
(B) TYPE: nucleic acid
(C) STRANDEDNESS: double
(D) TOPOLOGY: linear

(ii) MOLECULE TYPE: cDNA to mRNA

(iii) HYPOTHETICAL: N

(vii) IMMEDIATE SOURCE:
(B) CLONE: pLGC-x1

(ix) FEATURE:
(A) NAME/KEY: CDS
(B) LOCATION: 11..25

(D) OTHER INFORMATION:

(xi) SEQUENCE DESCRIPTION: SEQ ID NO:7:

```
CGATAAGCTT ATG CGA ATC TTC GTG                                        25
           Met Arg Ile Phe Val
             1               5
```

(2) INFORMATION FOR SEQ ID NO:8:

(i) SEQUENCE CHARACTERISTICS:
(A) LENGTH: 5 amino acids
(B) TYPE: amino acid
(D) TOPOLOGY: linear

(ii) MOLECULE TYPE: protein

(xi) SEQUENCE DESCRIPTION: SEQ ID NO:8:

```
Met Arg Ile Phe Val
  1             5
```

(2) INFORMATION FOR SEQ ID NO:9:

(i) SEQUENCE CHARACTERISTICS:
(A) LENGTH: 30 amino acids
(B) TYPE: amino acid
(D) TOPOLOGY: linear

(ii) MOLECULE TYPE: peptide

(iii) HYPOTHETICAL: N

(v) FRAGMENT TYPE: N-terminal
(vii) IMMEDIATE SOURCE:
(B) CLONE: pLGC-x1

(xi) SEQUENCE DESCRIPTION: SEQ ID NO:9:

```
Met Gln Ile Phe Val Lys Thr Leu Thr Gly Lys Thr Ile Thr Leu Glu
1               5                   10                  15

Val Glu Pro Xaa Asp Xaa Ile Xaa Asn Val Xaa Ala Xaa Ile
            20                  25                  30
```

(2) INFORMATION FOR SEQ ID NO:10:

(i) SEQUENCE CHARACTERISTICS:
(A) LENGTH: 471 base pairs
(B) TYPE: nucleic acid
(C) STRANDEDNESS: double
(D) TOPOLOGY: linear

(ii) MOLECULE TYPE: cDNA to mRNA

(vi) ORIGINAL SOURCE:
(H) CELL LINE: K562

(vii) IMMEDIATE SOURCE:
(B) CLONE: pLGA8

(ix) FEATURE:

(A) NAME/KEY: CDS
(B) LOCATION: 1..468
(D) OTHER INFORMATION: /product= "POLYUBIQUITIN"

(xi) SEQUENCE DESCRIPTION: SEQ ID NO:10:

```
ATG CAG ATT TTC GTG AAA ACC CTT ACG GGG AAG ACC ATC ACC CTC GAG      48
Met Gln Ile Phe Val Lys Thr Leu Thr Gly Lys Thr Ile Thr Leu Glu
  1               5                  10                  15

GTT GAA CCC TCG GAT ACG ATA GAA AAT GTA AAG GCC AAG ATC CAG GAT      95
Val Glu Pro Ser Asp Thr Ile Glu Asn Val Lys Ala Lys Ile Gln Asp
             20                  25                  30

AAG GAA GGA ATT CCT CCT GAT CAG CAG AGA CTG ATC TTT GCT GGC AAG     144
Lys Glu Gly Ile Pro Pro Asp Gln Gln Arg Leu Ile Phe Ala Gly Lys
         35                  40                  45

CAG CTG GAA GAT GGA CGT ACT TTG TCT GAC TAC AAT ATT CAA AAG GAG     192
Gln Leu Glu Asp Gly Arg Thr Leu Ser Asp Tyr Asn Ile Gln Lys Glu
     50                  55                  60

TCT ACT CTT CAT CTT GTG TTG AGA CTT CGT GGT GGT GCT AAG AAA AGG     240
Ser Thr Leu His Leu Val Leu Arg Leu Arg Gly Gly Ala Lys Lys Arg
 65                  70                  75                  80

AAG AAG AAG TCT TAC ACC ACT CCC AAG AAG AAT AAG CAC AAG AGA AAG     288
Lys Lys Lys Ser Tyr Thr Thr Pro Lys Lys Asn Lys His Lys Arg Lys
                 85                  90                  95

AAG GTT AAG CTG GCT GTC CTG AAA TAT TAT AAG GTG GAT GAG AAT GGC     336
Lys Val Lys Leu Ala Val Leu Lys Tyr Tyr Lys Val Asp Glu Asn Gly
            100                 105                 110

AAA ATT AGT CGC CTT CGT CGA GAG TGC CCT TCT GAT GAA TGT GGT GCT     384
Lys Ile Ser Arg Leu Arg Arg Glu Cys Pro Ser Asp Glu Cys Gly Ala
        115                 120                 125

GGG GTG TTT ATG GCA AGT CAC TTT GAC AGA CAT TAT TGT GGC AAA TGT     432
Gly Val Phe Met Ala Ser His Phe Asp Arg His Tyr Cys Gly Lys Cys
    130                 135                 140

TGT CTG ACT TCA TGT TTC AAC AAA CCA GAA GAC AAG TAA                 471
Cys Leu Thr Ser Cys Phe Asn Lys Pro Glu Asp Lys
145                 150                 155
```

(2) INFORMATION FOR SEQ ID NO:11:

(i) SEQUENCE CHARACTERISTICS:
(A) LENGTH: 156 amino acids
(B) TYPE: amino acid
(D) TOPOLOGY: linear

(ii) MOLECULE TYPE: protein

(xi) SEQUENCE DESCRIPTION: SEQ ID NO:11:

```
Met Gln Ile Phe Val Lys Thr Leu Thr Gly Lys Thr Ile Thr Leu Glu
  1               5                  10                  15

Val Glu Pro Ser Asp Thr Ile Glu Asn Val Lys Ala Lys Ile Gln Asp
             20                  25                  30
```

```
Lys Glu Gly Ile Pro Pro Asp Gln Gln Arg Leu Ile Phe Ala Gly Lys
        35                  40                  45

Gln Leu Glu Asp Gly Arg Thr Leu Ser Asp Tyr Asn Ile Gln Lys Glu
    50                  55                  60

Ser Thr Leu His Leu Val Leu Arg Leu Arg Gly Gly Ala Lys Lys Arg
65                  70                  75                  80

Lys Lys Lys Ser Tyr Thr Thr Pro Lys Lys Asn Lys His Lys Arg Lys
                85                  90                  95

Lys Val Lys Leu Ala Val Leu Lys Tyr Tyr Lys Val Asp Glu Asn Gly
            100                 105                 110

Lys Ile Ser Arg Leu Arg Arg Glu Cys Pro Ser Asp Glu Cys Gly Ala
        115                 120                 125

Gly Val Phe Met Ala Ser His Phe Asp Arg His Tyr Cys Gly Lys Cys
    130                 135                 140

Cys Leu Thr Ser Cys Phe Asn Lys Pro Glu Asp Lys
145                 150                 155
```

(2) INFORMATION FOR SEQ ID NO:12:

(i) SEQUENCE CHARACTERISTICS:
(A) LENGTH: 234 base pairs
(B) TYPE: nucleic acid
(C) STRANDEDNESS: double
(D) TOPOLOGY: linear

(ii) MOLECULE TYPE: cDNA to mRNA

(vi) ORIGINAL SOURCE:
(H) CELL LINE: K562

(vii) IMMEDIATE SOURCE:
(B) CLONE: pLGB12

(ix) FEATURE:
(A) NAME/KEY: CDS
(B) LOCATION: 1..231
(D) OTHER INFORMATION: /product= "POLYUBIQUITIN"

(xi) SEQUENCE DESCRIPTION: SEQ ID NO:12:

```
ATG CAA ATA TTC GTG AAG ACC CTG ACC GGC AAG ACC ATC ACT CTG GAG      48
Met Gln Ile Phe Val Lys Thr Leu Thr Gly Lys Thr Ile Thr Leu Glu
  1               5                  10                  15

GTG GAG CCC AGT GAC ACC ATC GAA AAT GTG AAG GCC AAG ATC CAA GAT      96
Val Glu Pro Ser Asp Thr Ile Glu Asn Val Lys Ala Lys Ile Gln Asp
             20                  25                  30

AAA GAA GGC ATC CCC CCC GAC CAG CAG AGG CTC ATC TTT GCA GGC AAG     144
Lys Glu Gly Ile Pro Pro Asp Gln Gln Arg Leu Ile Phe Ala Gly Lys
        35                  40                  45

CAG CTG GAA GAT GGC CGC ACT CTT TCT GAC TAC AAC ATC CAG AAA GAG     192
Gln Leu Glu Asp Gly Arg Thr Leu Ser Asp Tyr Asn Ile Gln Lys Glu
    50                  55                  60
```

```
TCG ACC CTG CAC CTG GTC CTG CGC CTG AGG GGT GGC TGT TAA                 234
Ser Thr Leu His Leu Val Leu Arg Leu Arg Gly Gly Cys
 65                 70                  75
```

(2) INFORMATION FOR SEQ ID NO:13:

(i) SEQUENCE CHARACTERISTICS:
(A) LENGTH: 77 amino acids
(B) TYPE: amino acid
(D) TOPOLOGY: linear

(ii) MOLECULE TYPE: protein

(xi) SEQUENCE DESCRIPTION: SEQ ID NO:13:

```
Met Gln Ile Phe Val Lys Thr Leu Thr Gly Lys Thr Ile Thr Leu Glu
 1               5                  10                  15

Val Glu Pro Ser Asp Thr Ile Glu Asn Val Lys Ala Lys Ile Gln Asp
            20                  25                  30

Lys Glu Gly Ile Pro Pro Asp Gln Gln Arg Leu Ile Phe Ala Gly Lys
        35                  40                  45

Gln Leu Glu Asp Gly Arg Thr Leu Ser Asp Tyr Asn Ile Gln Lys Glu
    50                  55                  60

Ser Thr Leu His Leu Val Leu Arg Leu Arg Gly Gly Cys
 65                 70                  75
```

(2) INFORMATION FOR SEQ ID NO:14:

(i) SEQUENCE CHARACTERISTICS:
(A) LENGTH: 690 base pairs
(B) TYPE: nucleic acid
(C) STRANDEDNESS: double
(D) TOPOLOGY: linear

(ii) MOLECULE TYPE: cDNA to mRNA

(vi) ORIGINAL SOURCE:
(H) CELL LINE: K562

(vii) IMMEDIATE SOURCE:
(B) CLONE: pLGC15

(ix) FEATURE:
(A) NAME/KEY: CDS
(B) LOCATION: 1..687
(D) OTHER INFORMATION: /product= "POLYUBIQUITIN"r

(xi) SEQUENCE DESCRIPTION: SEQ ID NO:14:

```
ATG CAG ATC TTC GTG AAA ACC CTT ACC GGC AAG ACC ATC ACC CTT GAG          48
Met Gln Ile Phe Val Lys Thr Leu Thr Gly Lys Thr Ile Thr Leu Glu
 1               5                  10                  15

GTG GAG CCC AGT GAC ACC ATC GAA AAT GTG AAG GCC AAG ATC CAG GAT          96
Val Glu Pro Ser Asp Thr Ile Glu Asn Val Lys Ala Lys Ile Gln Asp
            20                  25                  30

AAG GAA GGC ATT CCC CCC GAC CAG ACG AGG CTC ATC TTT GCA GGC AAG         144
Lys Glu Gly Ile Pro Pro Asp Gln Thr Arg Leu Ile Phe Ala Gly Lys
        35                  40                  45
```

```
CAG CTG GAA GAT GGC CGT ACT CTT TCT GAC TAC AAC ATC CAG AAG GAG         192
Gln Leu Glu Asp Gly Arg Thr Leu Ser Asp Tyr Asn Ile Gln Lys Glu
     50                  55                  60

TCG ACC CTG CAC CTG GTC CTG CGT CTG AGA GGT GGT ATG CAG ATC TTC         240
Ser Thr Leu His Leu Val Leu Arg Leu Arg Gly Gly Met Gln Ile Phe
 65                  70                  75                  80

GTG AAG ACC CTG ACC GGC AAG ACC ATC ACC CTG GAA GTG GAG CCC AGT         288
Val Lys Thr Leu Thr Gly Lys Thr Ile Thr Leu Glu Val Glu Pro Ser
                 85                  90                  95

GAC ACC ATC GAA AAT GTG AAG GCC AAG ATC CAG GAT AAA GAA GGC ATC         336
Asp Thr Ile Glu Asn Val Lys Ala Lys Ile Gln Asp Lys Glu Gly Ile
            100                 105                 110

CCT CCC GAC CAG CAG AGG CTC ATC TTT GCA GGC AAG CAG CTG GAA GAT         384
Pro Pro Asp Gln Gln Arg Leu Ile Phe Ala Gly Lys Gln Leu Glu Asp
        115                 120                 125

GGC CGC ACT CTT TCT GAC TAC AAC ATC CAG AAG GAG TCG ACC CTG CAC         432
Gly Arg Thr Leu Ser Asp Tyr Asn Ile Gln Lys Glu Ser Thr Leu His
    130                 135                 140

CTG GTC CTG CGT CTG AGA GGT GGT ATG CAG ATC TTC GTG AAG ACC CTG         480
Leu Val Leu Arg Leu Arg Gly Gly Met Gln Ile Phe Val Lys Thr Leu
145                 150                 155                 160

ACC GGC AAG ACC ATC ACT CTG GAG GTG GAG CCC AGT GAC ACC ATC GAA         528
Thr Gly Lys Thr Ile Thr Leu Glu Val Glu Pro Ser Asp Thr Ile Glu
                165                 170                 175

AAT GTG AAG GCC AAG ATC CAA GAT AAA GAA GGC ATC CCC CCC GAC CAG         576
Asn Val Lys Ala Lys Ile Gln Asp Lys Glu Gly Ile Pro Pro Asp Gln
            180                 185                 190

CAG AGG CTC ATC TTT GCA GGC AAG CAG CTG GAA GAT GGC CGC ACT CTT         624
Gln Arg Leu Ile Phe Ala Gly Lys Gln Leu Glu Asp Gly Arg Thr Leu
        195                 200                 205

TCT GAC TAC AAC ATC CAG AAA GAG TCG ACC CTG CAC CTG GTC CTG CGC         672
Ser Asp Tyr Asn Ile Gln Lys Glu Ser Thr Leu His Leu Val Leu Arg
    210                 215                 220

CTG AGG GGT GGC TGT TAA                                                 690
Leu Arg Gly Gly Cys
225
```

(2) INFORMATION FOR SEQ ID NO:15:

(i) SEQUENCE CHARACTERISTICS:
(A) LENGTH: 229 amino acids
(B) TYPE: amino acid
(D) TOPOLOGY: linear

(ii) MOLECULE TYPE: protein

(xi) SEQUENCE DESCRIPTION: SEQ ID NO:15:

```
Met Gln Ile Phe Val Lys Thr Leu Thr Gly Lys Thr Ile Thr Leu Glu
  1               5                  10                  15

Val Glu Pro Ser Asp Thr Ile Glu Asn Val Lys Ala Lys Ile Gln Asp
             20                  25                  30
```

```
Lys Glu Gly Ile Pro Pro Asp Gln Thr Arg Leu Ile Phe Ala Gly Lys
        35                  40                  45

Gln Leu Glu Asp Gly Arg Thr Leu Ser Asp Tyr Asn Ile Gln Lys Glu
    50                  55                  60

Ser Thr Leu His Leu Val Leu Arg Leu Arg Gly Gly Met Gln Ile Phe
65                  70                  75                  80

Val Lys Thr Leu Thr Gly Lys Thr Ile Thr Leu Glu Val Glu Pro Ser
                85                  90                  95

Asp Thr Ile Glu Asn Val Lys Ala Lys Ile Gln Asp Lys Glu Gly Ile
            100                 105                 110

Pro Pro Asp Gln Gln Arg Leu Ile Phe Ala Gly Lys Gln Leu Glu Asp
        115                 120                 125

Gly Arg Thr Leu Ser Asp Tyr Asn Ile Gln Lys Glu Ser Thr Leu His
    130                 135                 140

Leu Val Leu Arg Leu Arg Gly Gly Met Gln Ile Phe Val Lys Thr Leu
145                 150                 155                 160

Thr Gly Lys Thr Ile Thr Leu Glu Val Glu Pro Ser Asp Thr Ile Glu
                165                 170                 175

Asn Val Lys Ala Lys Ile Gln Asp Lys Glu Gly Ile Pro Pro Asp Gln
            180                 185                 190

Gln Arg Leu Ile Phe Ala Gly Lys Gln Leu Glu Asp Gly Arg Thr Leu
        195                 200                 205

Ser Asp Tyr Asn Ile Gln Lys Glu Ser Thr Leu His Leu Val Leu Arg
    210                 215                 220

Leu Arg Gly Gly Cys
225
```

## Claims

1. A method of assaying anti-ubiquitin antibody which comprises allowing anti-ubiquitin antibody in a sample to bind to polyubiquitin supported on a solid phase, then allowing a labeled anti-human immunoglobulin specific antibody to bind to the bound anti-ubiquitin antibody, and assaying the label on the anti-human immunoglobulin specific antibody.

2. A assay method as claimed in Claim 1, wherein said polyubiquitin comprises three repetitions of the peptide sequence shown under SEQ ID NO: 1.

3. An assay method as claimed in Claim 1, wherein said label is selected from the group consisting of enzymes, biotin, chemiluminescent substances and radioactive compounds.

4. An assay method as claimed in Claim 3, wherein the label is an enzyme selected from the group consisting of peroxidase, urease, alkaline phosphatase and $\beta$-galactosidase.

5. An anti-ubiquitin antibody assay kit which comprises a solid support carrying polyubiquitin, a labeled anti-human immunoglobulin specific antibody, and an assay system for the label on said specific antibody.

6. An anti-ubiquitin antibody assay kit as claimed in Claim 5, wherein said polyubiquitin comprises three repetitions of the peptide sequence shown under SEQ ID NO: 1.

**7.** A method for identifying a patient with systemic lupus erythematosus comprising the steps of:
(a) contacting a sample to polyubiquitin; and
(b) detecting the formation of a polyubiquitin/anti-ubiquitin antibody complex.

**8.** A method for identifying a patient as claimed in Claim 7, wherein said polyubiquitin comprises three repetitions of the peptide sequence shown under SEQ ID NO: 1.

**9.** A method for indentifying a patient with systemic lupus erythematosus comprising the steps of:
(a) supporting polyubiquitin on a solid phase;
(b) contacting a sample to said solid phase;
(c) contacting a labeled anti-human immunoglobulin specific antibody to said solid phase; and
(d) detecting the formation of an anti-ubiquitin antibody/anti-human immunoglobulin specific antibody complex on said solid phase by means of said label.

**10.** A method for identifying a patient as claimed in Claim 9, wherein said polyubiquitin comprises three repetitions of the peptide sequence shown under SEQ ID NO: 1.

Fig. 1

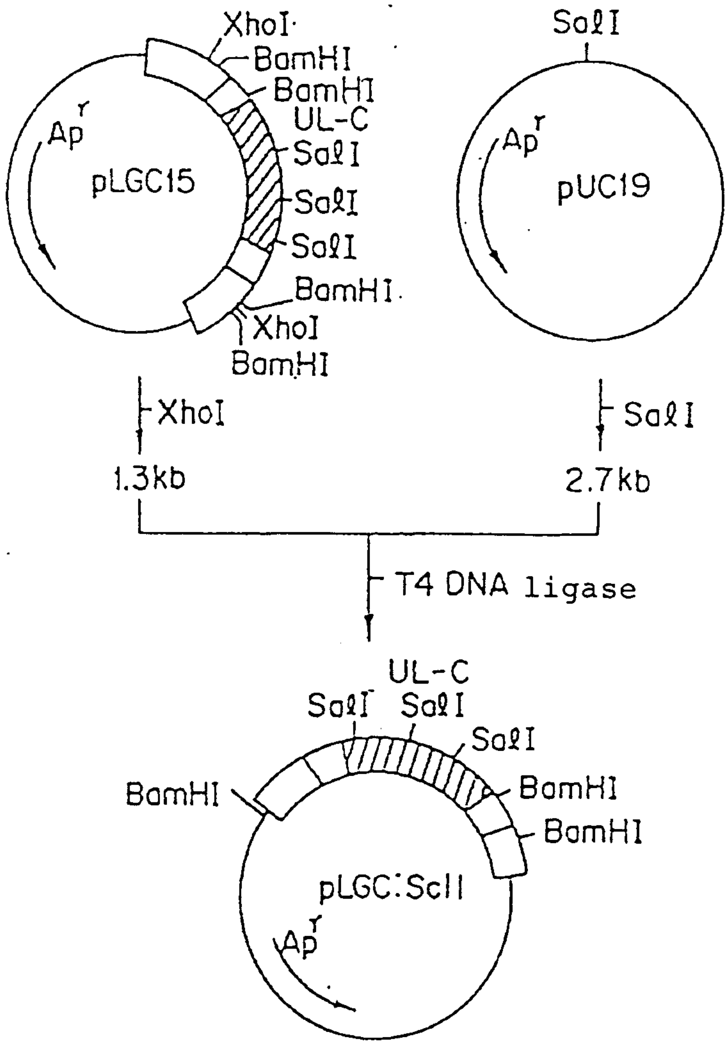


UL-C: DNA region indicated by SEQ ID NO: 10

Fig. 2

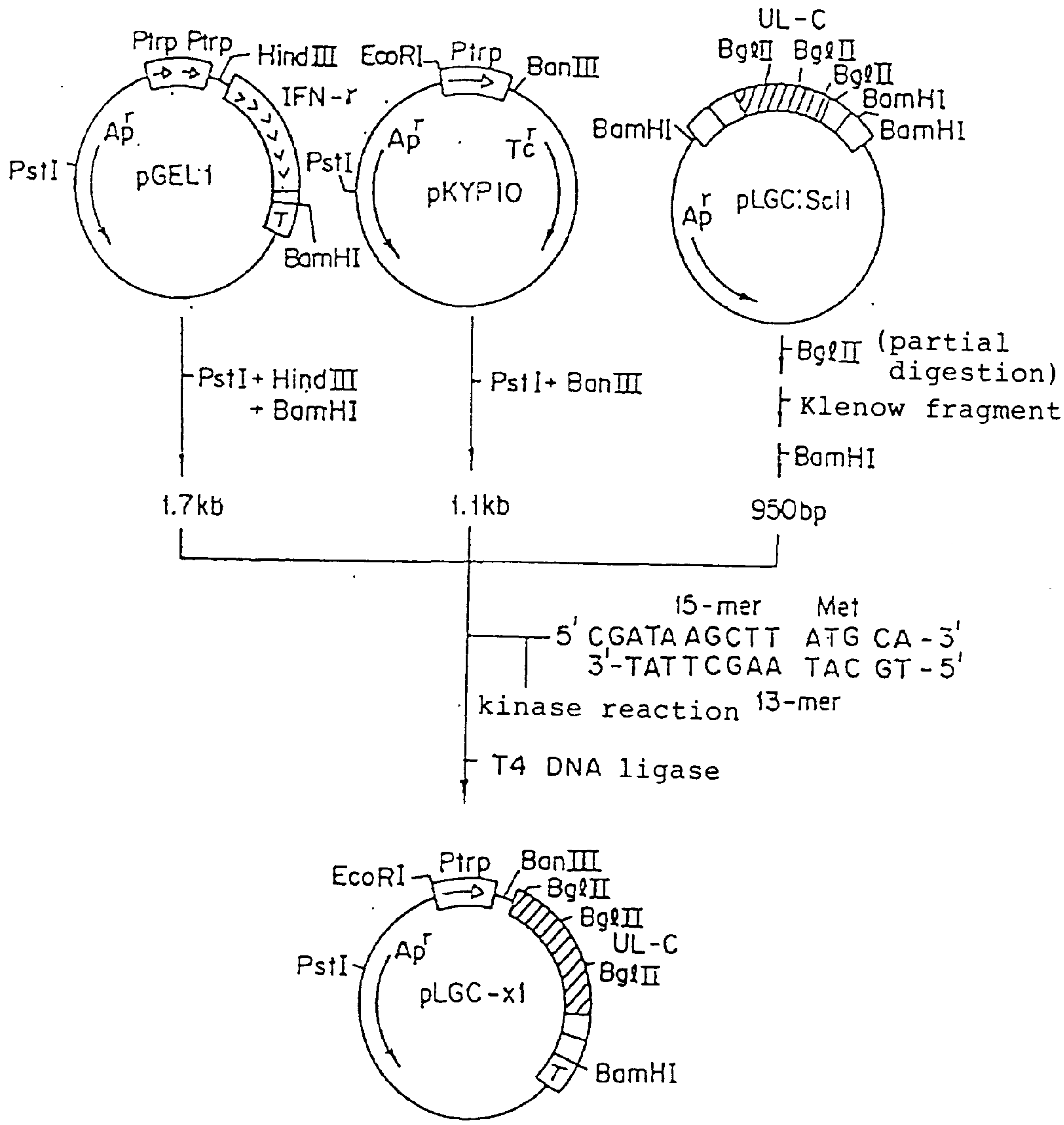
Ptrp Ptrp
HindIII
IFN-γ
Ap^r
PstI
pGEL1
T
BamHI
EcoRI
Ptrp
BanIII
Ap^r
Tc^r
PstI
pKYP10
UL-C
BglII BglII
BglII
BamHI
BamHI
BamHI
Ap^r
pLGC:ScII
PstI + HindIII
+ BamHI
PstI + BanIII
BglII (partial digestion)
Klenow fragment
BamHI
1.7kb
1.1kb
950bp
15-mer Met
5' CGATA AGCTT ATG CA-3'
3'-TATTCGAA TAC GT-5'
kinase reaction 13-mer
T4 DNA ligase
EcoRI
Ptrp
BanIII
BglII
BglII
UL-C
BglII
PstI
Ap^r
pLGC-x1
T
BamHI

Fig. 3

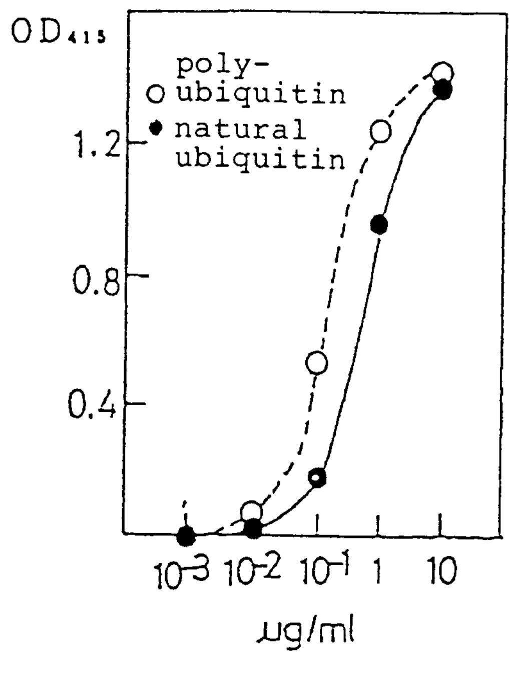


Rabbit anti-ubiquitin polyclonal antibody

Fig. 4

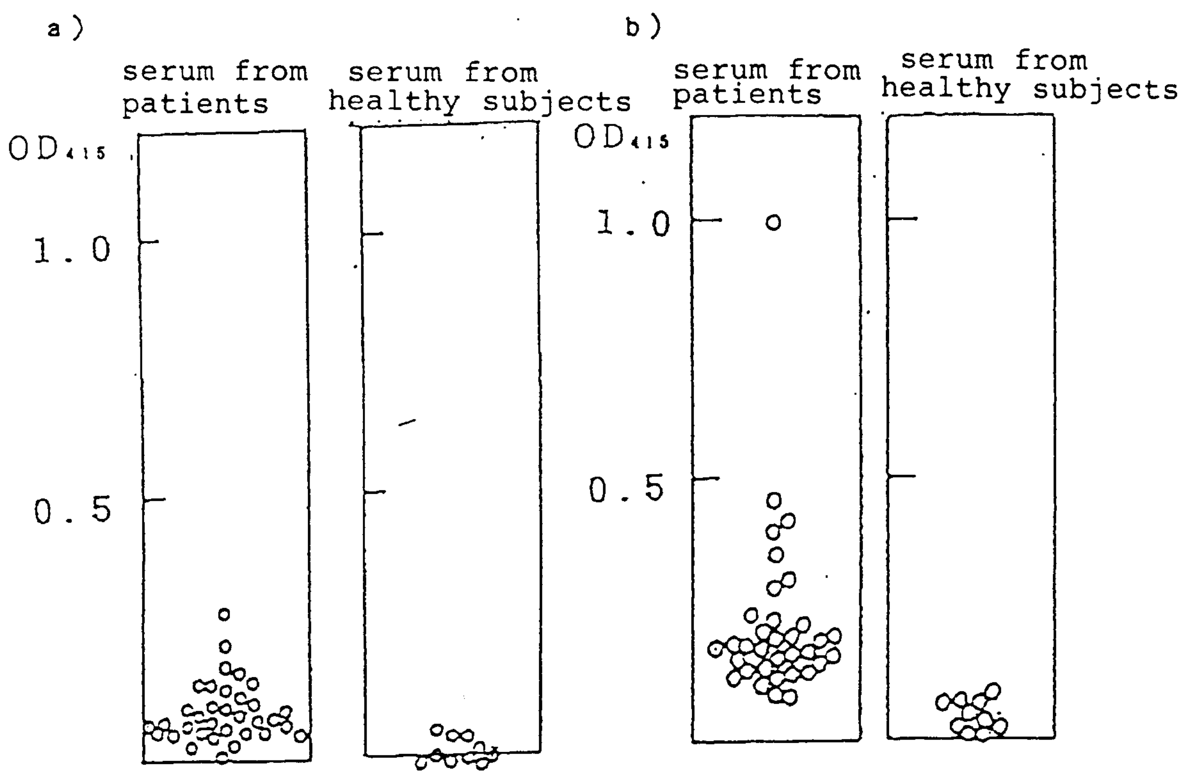


a) ubiquitin was used as antigenic protein.

b) polyubiquitin was used as antigenic protein.

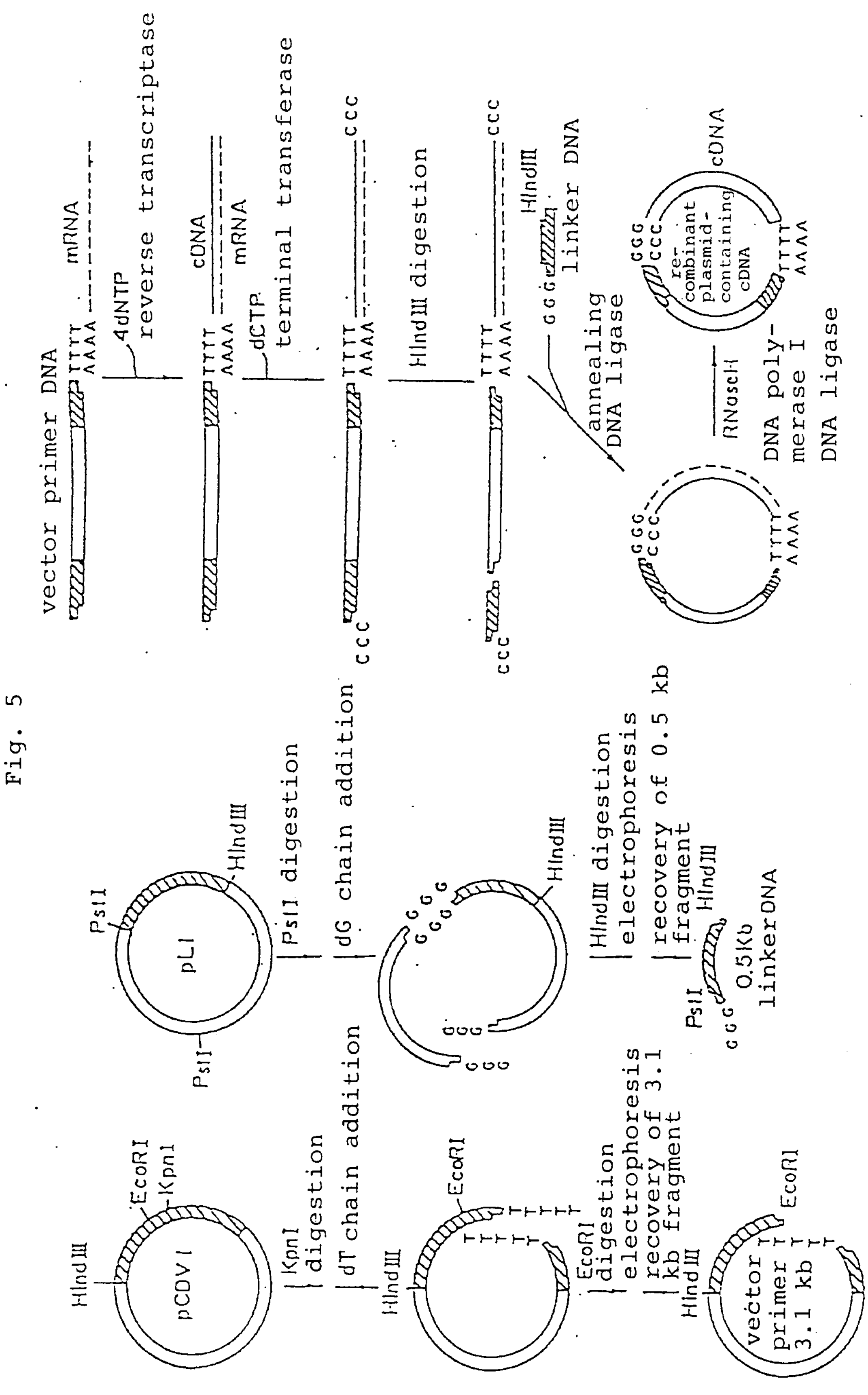
pCDV1
HindIII
EcoRI
KpnI
KpnI digestion
dT chain addition
EcoRI digestion
electrophoresis
recovery of 3.1 kb fragment
vector primer 3.1 kb
pL1
PstI
PstI digestion
dG chain addition
HindIII digestion
electrophoresis
recovery of 0.5 kb fragment
0.5kb linker DNA
vector primer DNA
TTTT
AAAA
mRNA
4dNTP
reverse transcriptase
cDNA
dCTP
terminal transferase
CCC
HindIII digestion
linker DNA
GGG
annealing
DNA ligase
recombinant plasmid-containing cDNA
RNaseH
DNA polymerase I
DNA ligase

Fig. 5

Fig. 6

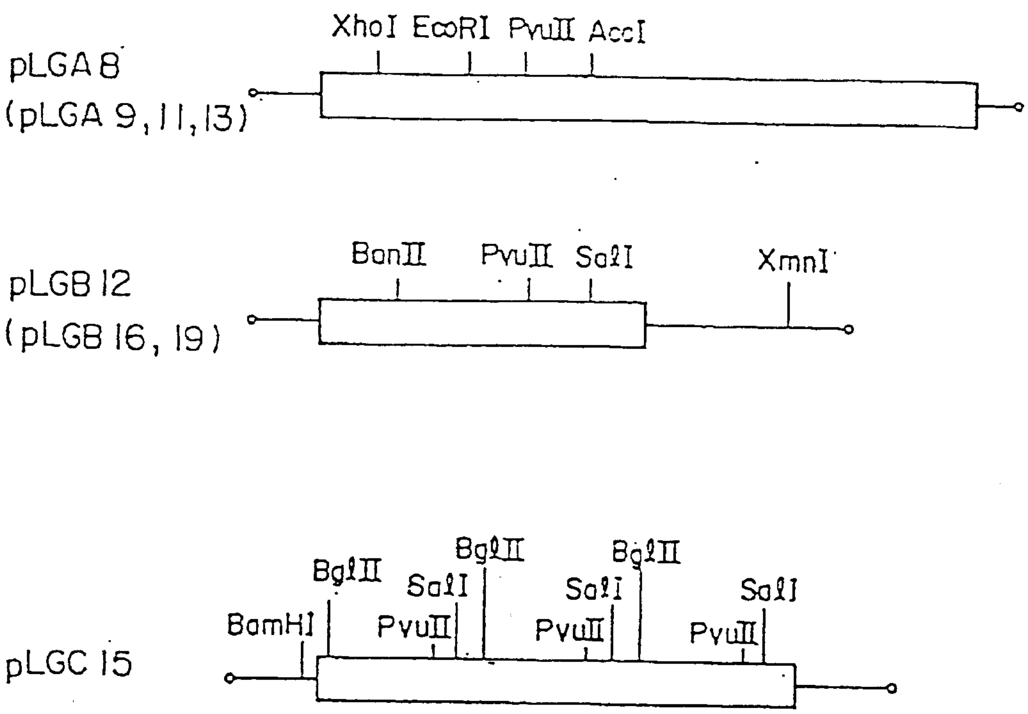
pLGA 8
(pLGA 9, 11, 13)
XhoI EcoRI PvuII AccI
pLGB 12
(pLGB 16, 19)
BanII PvuII SalI XmnI
pLGC 15
BamHI BglII PvuII SalI BglII PvuII SalI BglII PvuII SalI

Fig. 7

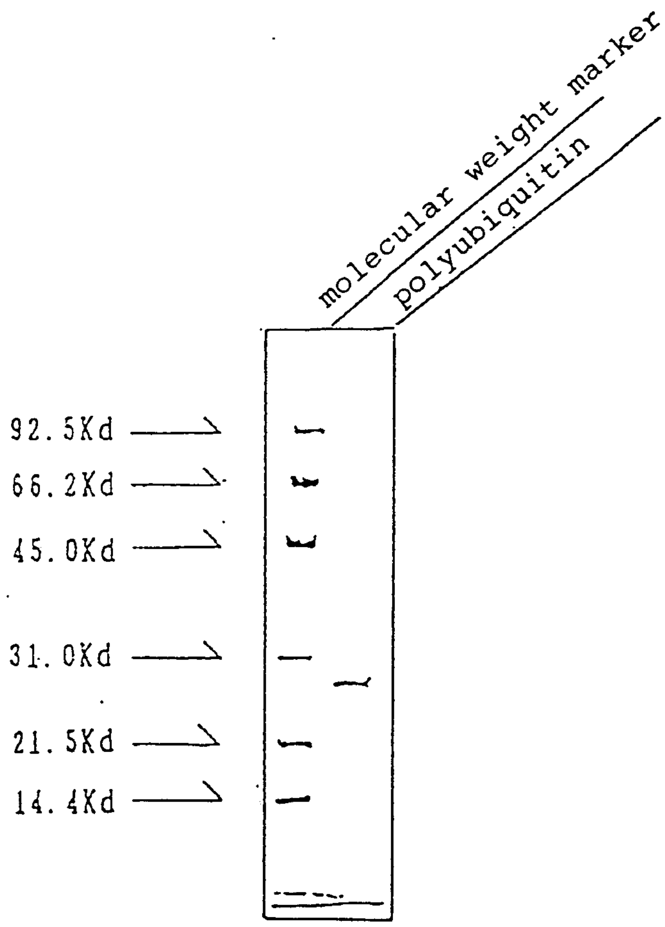
molecular weight marker
polyubiquitin
92.5Kd
66.2Kd
45.0Kd
31.0Kd
21.5Kd
14.4Kd